# EUROPEAN PATENT APPLICATION

(11) **EP 4 282 443 A1**
(43) Date of publication of application: **29.11.2023**
(21) Application number: 21928824.8
(22) Date of filing: 27.10.2021
(51) Int. Cl.: A61L 24/04, A61L 31/04, A61L 26/00

(54) **MEDICAL TISSUE ADHESIVE AND PREPARATION METHOD THEREFOR**

(30) Priority: 04.03.2021 CN 202110239436
(71) Applicant: Haining Zhuluoji Biotechnology Co., Ltd., Haining, Zhejiang 314400 (CN)
(72) Inventor: QIU, Lingxiao, Zhejiang 314400 (CN); CHEN, Xiaojie, Zhejiang 314400 (CN)
(74) Representative: Taor, Simon Edward William
(86) International application number: PCT/CN2021/126764
(87) International publication number: WO 2022/183750

(57) **Abstract**

The invention provides a medical tissue adhesive and a preparation method thereof, belonging to the adhesive field. The medical tissue adhesive provided by the invention includes: a functional component, an assistant crosslinker and a dispersing agent, wherein the functional component is a polymer chain modified by tissue adhesive group or a micro nano particle modified by tissue-adhering functional groups, and the tissue-adhering functional groups includes: o-nitrobenzyl photoplate group, active ester group or carbonyl group. The medical tissue adhesive provided by the invention can not only use the dispersing agent to expel the tissue fluid or blood on the tissue surface, so that the adhesive molecular segment in the functional component can quickly form a chemical bond with the amino group on the tissue surface to form a strong tissue adhesive force, but also can trigger the adhesive molecular segment in the active ingredient to react with the amino group on the assistant crosslinker, so that the medical tissue adhesive can be quickly gelled and solidified as a whole. Therefore, it has good bleeding performance, hemostatic performance and tissue adhesion performance.

## Description

### Technical Field

This present invention relates to the technical field of adhesives, be specifically related to a kind of medical tissue adhesive and the preparation method thereof.

### Background technology

Medical adhesives mainly include tissue adhesives, hemostatic agents, and tissue sealants, which have been widely used in clinical operations. Tissue adhesive can be broadly defined as any substance with in-situ polymerization properties, which can make tissue and tissue or tissue and non-tissue surface adhesion, control bleeding (hemostatic agent), and act as a barrier preventing gas and liquid leakage (Sealants). The ideal biomedical adhesive needs to possess the following characteristics, biocompatibility, biodegradability, mechanical compliance with underlying tissues, acceptable swelling index, and storage stability. Specifically, it should be safe, non-toxic, and easy to sterilize and prepare; it should have fluid properties and easy to be used on wounds; it should be cured quickly in a physiological environment, reducing bleeding and operation time; it should have excellent tissue adhesion performance and remain stable within a specific time; it also needs to maintain the original mechanical properties during the healing process; lastly, the degradation time should be appropriate, and the degradation products need to be non-toxic.

At present, the commonly used commercial medical adhesives generally consists of two parts, the functional component part and the cross-linking agent part, which are separately dissolved into solutions before usage. And then the functional component and the cross-linking agent are mixed through the blending injection head and injected into the bleeding Location. However, this will cause inconvenience in clinical use and uncontrollable gelation speed (too fast or too slow to gel), which may lead to wash-off of adhesives by a large amount of blood on the surface of the tissue, resulting in unsatisfactory hemostatic effect. All these shortcomings restrict this kind of adhesives only being applied to hemostasis with a small amount of bleeding.

Patent CN201910049714 .4 uses water-immiscible castor oil and other hydrophobic organic solvents as dispersing agents to disperse the functional components and auxiliary crosslinking agents, and increase the probability of the functional components in contact with the tissue surface through the expelling blood effect of hydrophobic dispersion, in order to obtain the ability of adhering to the surface of tissues with a large amount of blood. However, as the functional component and auxiliary crosslinking agent are wrapped by a large amount of hydrophobic dispersing agent, it is difficult to for them to obtain enough water to dissolve and crosslink subsequently which leads to slow gel formation and poor gel strength, and failure of rapid hemostasis and strong tissue adhesion.

### Summary of the invention

The present invention is made to solve the problems, and aims to provide a method of producing medical tissue adhesive.

The present invention provides a medical tissue adhesive, which is used to bond biological tissues, and contains a functional component, comprising polymer chains or micro/nano particles modified with tissue-adhering functional groups; an assistant crosslinker, comprising chemicals that crosslink or stimulate crosslinking of the tissue-adhering functional groups; and a dispersing agent, comprising a compound which can facilitate contact between the functional components and the biological tissue upon triggered by water-containing fluid.

In the medical tissue adhesive, provided by the present invention, the tissue-adhering functional group comprises: o-Nitrobenzyl photo-trigger group, active ester group, isocyanate group, isothiocyanate group, epoxide group, cyclic carbonate group, cyclic thiocarbonate group, active carbonyl group, or active double bond group, wherein the o-Nitrobenzyl photo-trigger group is expressed by structural formula (I) and structural formula (II) below:

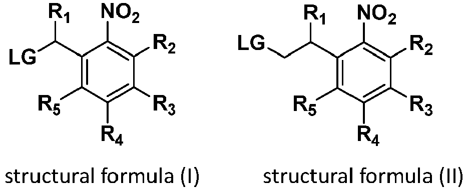

In formula (I) and (II), LG is selected from halogen atom, O-R', S-R', or NH-R'; wherein R' is one of the following groups: hydrogen, alkyl group, ether group, thioether group, carbonyl group, ester group, thioester group, amide group, sulfate group, sulphonate group, or phosphonate group;
R1 is one of the following groups: hydrogen, halogen atom, hydroxy group, mercapto group, amino group, nitro group, cyano group, aldehyde group, carbonyl group, ester group, amide group, thioester group, sulfate group, sulphonate group, phosphonate group, sulfuryl group, sulfoxide group, aryl group, alkyl group, or modified alkyl group.

One or more of R2, R3, R4, R5 are linked with the polymer chains or the micro/nano particles.

R2, R3, R4 or R5before linked with the polymer chains or the micro/nano particles is one of the following groups: hydrogen, halogen atom, hydroxy group, mercapto group, amino group, nitro group, cyano group, aldehyde group, carbonyl group, carboxyl group, ester group, thioester group, isocyanate group, isothiocyanate group, epoxide group, carbonate group, thiocarbonate group, cyclic carbonate group, cyclic thiocarbonate group, amide group, sulfate group, sulphonate group, phosphonate group, sulfuryl group, sulfoxide group, aryl group, alkyl group, or modified alkyl group.

The active ester group is a succinimide active ester group expressed by structural formula (III) or a triazole active ester group expressed by structural formula (IV) below:

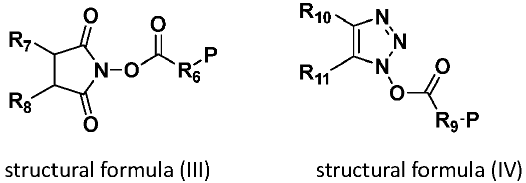

The succinimide active ester group is linked via R6 with the polymer chains or the micro/nano particles denoted as P.

In structural formula (III), R6 is directly linked with the polymer chains or the micro/nano particles, or indirectly linked with the polymer chains or the micro/nano particles via one of the following modified end groups: ether bond, thioether bond, carbonyl group, ester group, thioester group, amide group, sulfate group, sulphonate group, phosphonate group, sulfuryl group, sulfoxide group, aryl group, alkyl group, or modified alkyl group.

R7, R8 are either cyclized with each other or each selected from hydrogen, halogen atom, hydroxy group, mercapto group, amino group, nitro group, cyano group, aldehyde group, carbonyl group, ester group, thioester group, isocyanate group, isothiocyanate group, epoxide group, carbonate group, thiocarbonate group, cyclic carbonate group, cyclic thiocarbonate group, amide group, sulfate group, sulphonate group, phosphonate group, sulfuryl group, sulfoxide group, aryl group, alkyl group, or modified alkyl group,
The thiazole active ester group is linked via R9 with the polymer chains or the micro/nano particles denoted as P.

In structural formula (IV), R9 is directly linked with the polymer chains or the micro/nano particles, or indirectly linked with the polymer chains or the micro/nano particles via one of the following modified end groups: ether bond, thioether bond, carbonyl group, ester group, thioester group, amide group, sulfate group, sulphonate group, phosphonate group, sulfuryl group, sulfoxide group, aryl group, alkyl group, or modified alkyl group.

R10, R11 are either cyclized with each other or each selected from hydrogen, halogen atom, hydroxy group, mercapto group, amino group, nitro group, cyano group, aldehyde group, carbonyl group, ester group, thioester group, isocyanate group, isothiocyanate group, epoxide group, carbonate group, thiocarbonate group, cyclic carbonate group, cyclic thiocarbonate group, amide group, sulfate group, sulphonate group, phosphonate group, sulfuryl group, sulfoxide group, aryl group, alkyl group, or modified alkyl group.

The isocyanate group is expressed by structural formula (V) below:

**O=C=N-R₁₂** structural formula (V)

In structural formula (V), R12 is linked with the polymer chains or the micro/nano particles, and R12 before linked is one of the following end groups: end amino group, hydroxy group, mercapto group, halogen atom, acyl halide group, acid anhydride group, carboxyl group, carboxylate group, active ester group, isocyanate group, isothiocyanate group, epoxide group, cyclic carbonate group, cyclic thiocarbonate group, carbonate group, aryl group modified with active double bond group or active carbonyl group, heteroaromatic group, alkyl group, or modified alkyl group.

The isothiocyanate group is expressed by structural formula (VI) below:

**S=C=N-R₁₃** structural formula (VI)

In structural formula (VI), R13 is further linked with the polymer chains or the micro/nano particles, and R13 before linked is one of the following end groups: end amino group, hydroxy group, mercapto group, halogen atom, acyl halide group, acid anhydride group, carboxyl group, carboxylate group, active ester group, isocyanate group, isothiocyanate group, epoxide group, cyclic carbonate group, cyclic thiocarbonate group, carbonate group, aryl group modified with active double bond group or active carbonyl group, heteroaromatic group, alkyl group, or modified alkyl group.

The epoxide group is expressed by structural formula (VII) below:

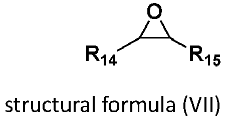

In structural formula (VII), R14 that is further linked with the polymer chains or the micro/nano particles, and R14 before linked is one of the following end groups: end amino group, hydroxy group, mercapto group, halogen atom, acyl halide group, acid anhydride group, carboxyl group, carboxylate group, active ester group, isocyanate group, isothiocyanate group, epoxide group, cyclic carbonate group, cyclic thiocarbonate group, carbonate group, aryl group modified with active double bond group or active carbonyl group, heteroaromatic group, alkyl group, or modified alkyl group.

In structural formula (VII), R15 is either cyclized with R14 or selected from hydrogen, halogen atom, hydroxy group, mercapto group, amino group, nitro group, cyano group, aldehyde group, carbonyl group, carboxyl group, ester group, thioester group, isocyanate group, isothiocyanate group, epoxide group, cyclic carbonate group, cyclic thiocarbonate group, carbonate group, thiocarbonate group, amide group, sulfate group, sulphonate group, phosphonate group, sulfuryl group, sulfoxide group, aryl group, alkyl group, or modified alkyl group.

The cyclic carbonate group is expressed by structural formula (VIII) below:

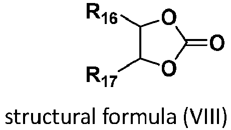

In structural formula (VIII), R16 is further linked with the polymer chains or the micro/nano particles, and R16 before linked is one of the following end groups: end amino group, hydroxy group, mercapto group, halogen atom, acyl halide group, acid anhydride group, carboxyl group, carboxylate group, active ester group, isocyanate group, isothiocyanate group, epoxide group, cyclic carbonate group, cyclic thiocarbonate group, carbonate group, aryl group modified with active double bond group or active carbonyl group, heteroaromatic group, alkyl group, or modified alkyl group.

In structural formula (VIII), R17 is either cyclized with R16 or selected from hydrogen, halogen atom, hydroxy group, mercapto group, amino group, nitro group, cyano group, aldehyde group, carbonyl group, carboxyl group, ester group, thioester group, isocyanate group, isothiocyanate group, epoxide group, cyclic carbonate group, cyclic thiocarbonate group, carbonate group, thiocarbonate group, amide group, sulfate group, sulphonate group, phosphonate group, sulfuryl group, sulfoxide group, aryl group, alkyl group, or modified alkyl group.

The cyclic thiocarbonate group is one of the seven chemicals expressed by structural formula (IX) below:

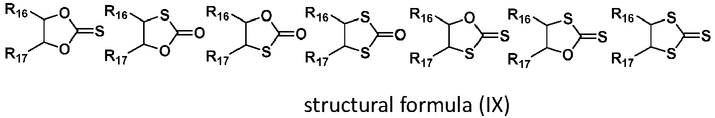

The active carbonyl group is expressed by structural formula (X) below:

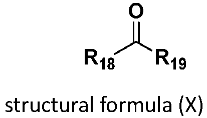

In structural formula (X), R18 is further linked with the polymer chains or the micro/nano particles, and R18 before linked is one of the following end groups: end amino group, hydroxy group, mercapto group, halogen atom, acyl halide group, acid anhydride group, carboxyl group, carboxylate group, active ester group, isocyanate group, isothiocyanate group, epoxide group, cyclic carbonate group, cyclic thiocarbonate group, carbonate group, aryl group modified with active double bond group or active carbonyl group, heteroaromatic group, alkyl group, or modified alkyl group.

In structural formula (X), R19 is either cyclized with R18 or selected from hydrogen, halogen atom, hydroxy group, mercapto group, amino group, nitro group, cyano group, aldehyde group, carbonyl group, carboxyl group, ester group, thioester group, isocyanate group, isothiocyanate group, epoxide group, cyclic carbonate group, cyclic thiocarbonate group, carbonate group, thiocarbonate group, amide group, sulfate group, sulphonate group, phosphonate group, sulfuryl group, sulfoxide group, aryl group, alkyl group, or modified alkyl group.

The active double bond is expressed by structural formula (XI) below:

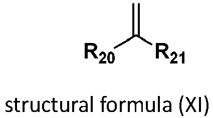

In structural formula (XI), R20 is further linked with the polymer chains or the micro/nano particles, and R18 before linked is one of the following end groups: end amino group, hydroxy group, mercapto group, halogen atom, acyl halide group, acid anhydride group, carboxyl group, carboxylate group, active ester group, isocyanate group, isothiocyanate group, epoxide group, cyclic carbonate group, cyclic thiocarbonate group, carbonate group, aryl group modified with active double bond group or active carbonyl group, heteroaromatic group, alkyl group, or modified alkyl group.

In structural formula (XI), R21 is either cyclized with R20 or selected from hydrogen, halogen atom, hydroxy group, mercapto group, amino group, nitro group, cyano group, aldehyde group, carbonyl group, carboxyl group, ester group, thioester group, isocyanate group, isothiocyanate group, epoxide group, cyclic carbonate group, cyclic thiocarbonate group, carbonate group, thiocarbonate group, amide group, sulfate group, sulphonate group, phosphonate group, sulfuryl group, sulfoxide group, aryl group, alkyl group, or modified alkyl group.

The medical tissue adhesive provided by the present invention also has the feature: wherein, the dispersing agent is a liquid dispersing agent, comprising one of or a mixture of polyhydric alcohols, liquid polyethylene glycol and its derivatives.

The medical tissue adhesive provided by the present invention also has the following characteristics: the polymer chain is natural saccharide chemical, hydrophilic polymer, or water-soluble synthetic polymer, while the micro/nano particle is a nanoparticle or microparticle of a polymer chain.

In the medical tissue adhesive provided by the present invention, it also has such characteristics: among them, the natural saccharide chemical is one or a mixture of hyaluronic acid, carboxymethyl cellulose, methyl cellulose, hydroxyethyl cellulose, hydroxypropyl cellulose, alginate, dextran, agarose, heparin, chondroitin sulfate, ethylene glycol chitosan, propylene glycol chitosan, chitosan lactate, carboxymethyl chitosan, and quaternary ammonium salt of chitosan.

In the medical tissue adhesive provided by the present invention, it also has such characteristics: the hydrophilic polymer or water-soluble synthetic polymer is one or a mixture of linear PEG, multi-arm PEG, polyethyleneimine, dendrimer, synthetic polypeptide, polyurethane, polylysine, polyglutamate, polyacrylate, polymethylacrylic acid, polymethacrylate, polyacrylamide, polymethacrylamide, polyvinyl alcohol, polyvinylpyrrolidone.

The medical tissue adhesive provided by the present invention also has the feature that the assistant crosslinker is a compound bearing amino group, or micro-nanoparticle consisting of compound bearing amino group.

The medical tissue adhesive provided by the present invention also has the feature that the compound bearing amino group is one of the following materials: hydrophilic or water-soluble animal/plant protein, collagen, serum protein, silk fibroin, elastin, and protein, degradants, polyethyleneimine, dendrimers, synthetic peptides, polylysine, polyarginine, polyhistidine, amino-terminated two-arm polyethylene glycol, amino-terminated multi-arm polyethylene glycol or amino blocked polyurethanes.

In the medical tissue adhesive provided by the present invention, it also has the following characteristics: wherein the general formula of the polyhydric alcohols is CₙH₂ₙ₋ₘ₊₂(OH)ₘ, wherein n is positive integer greater than or equal to 2, m is positive integer less than or equal to n, and the polyethylene glycol and its derivative is one of or a mixture of linear PEG, branched PEG, multi-arm polyethylene glycol homopolymer and copolymer, end-modified PEG, and its ionization products.

The medical tissue adhesive provided by the present invention also has the feature that the assistant crosslinker is a compound bearing amino group, or micro-nanoparticle consisting of compound bearing amino group.

In the medical tissue adhesive provided by the present invention, it also has the following characteristics: wherein the dispersing agent is solid dispersing agent, wherein the solid dispersing agent melts and evenly disperses the functional component and the assistant crosslinker upon heating, yet does not react with the functional component and the assistant crosslinker. The dispersing agent is one of or a mixture of solid PEG, its derivatives, and saccharide chemicals.

In the medical tissue adhesive provided by the present invention, it also has the feature: the solid polyethylene glycol and its derivative is one of or a mixture of linear PEG, branched PEG, multi-arm polyethylene glycol homopolymer and copolymer, end-modified PEG, and its ionization products.

In the medical tissue adhesive provided by the present invention, it also has the following characteristics: wherein the assistant crosslinker is the chemicals that react with the tissue-adhering functional groups, the assistant crosslinker is one of or a mixture of chemicals that bearing functional group comprising amino group, carboxyl group, mercapto group, or hydroxy group.

The medical tissue adhesive provided by the present invention also has the following characteristics:
The chemicals that bearing amino group is one of or a mixture of polyethyleneimine, polylysine, polyarginine, polyhistine, gelatin, collagen, elastin, fibroin, chitosan and its derivatives or copolymer, linear polyethylene glycol end-capped with amino group, multi-arm polyethylene glycol end-capped with amino group, and polyurethane end-capped with amino group; the chemicals that bearing carboxyl group is one of or a mixture of hyaluronic acid, carboxymethyl chitosan, carboxymethyl cellulose, carboxymethyl starch, alginate, chondroitin sulfate, heparin, polyuronic acid, polyglutamic acid, polyaspartic acid, gelatin, collagen, fibroin, and their derivatives or copolymer; the chemicals that bearing mercapto group is one or a mixture of polycystaine, gelatin, collagen, and their derivatives or copolymer; the chemicals that bearing hydroxy group is one or a mixture of hyaluronic acid, chitosan, agarose, cellulose, starch, alginate, chondroitin sulfate, heparin, polyuronic acid, cyclodextrin, polyserine, gelatin, collagen, fibroin, polyvinyl alcohol, and their derivatives or copolymer.

In the medical tissue adhesive provided by the present invention, it also has the following characteristics: wherein the assistant crosslinker is a chemical that stimulates self-crosslink between the tissue-adhering functional group, and the assistant crosslinker is peroxide or reductant.

In the medical tissue adhesive provided by the present invention, it also has the following characteristics: wherein the peroxide is one or more of the following groups: alkyl peroxide (ROOH), dialkyl peroxide (ROOR'), diacyl peroxide (RCOOOOCR'), peroxy ester (RCOOOR'), peroxycarbonate (ROCOOOOCOR') and ketone peroxide [R2C(OOH)2], furthermore benzoyl peroxide, peroxide tert-butyl, cyclohexanone peroxide, methyl ethyl ketone peroxide, hydrogen peroxide, persulfate, hydrogen persulfate, potassium persulfate and ammonium persulfate are preferred to choose.

In the medical tissue adhesive provided by the present invention, it also has such characteristics: wherein the reductant is one or more of the following materials: sulfite, bisulfite, ferrous salt, naphthenate, tertiary amine compound, mercaptan, furthermore sodium sulfite, sodium bisulfite, ferrous chloride, ferrous sulfate, cobalt naphthenate, N,N-dimethylaniline and tetraethylethylenediamine are perferable.

The medical tissue adhesive provided by the present invention also has the characteristic that the mass ratio of the functional component, the assistant crosslinker, and the dispersing agent is 1:(0.01^{~}10):(0.1^{~}30)..

The present invention provides a method for preparing a kind of medical tissue adhesive, which has the above-mentioned characteristics, including the following steps: grinding functional components and auxiliary crosslinking agents into powder, adding dispersing agent, and mixing them evenly.

The mechanism of the medical tissue adhesive provided by the present invention is as follows: When the medical tissue adhesive comes into contact with the wet tissue surface containing tissue fluid or blood, the dispersing agent in the medical tissue adhesive absorbs water to dissolve, and the dissolved dispersing agent squeezes out the tissue fluid or blood on the tissue surface, allowing the functional components and the assistant crosslinker agent fully contacting the surface of the tissue. Subsequently, the adhesive molecular fragments in the functional components quickly form chemical bonds with the amino, carboxyl or hydroxyl groups on the tissue surface with strong tissue adhesion force. At the same time, the medical tissue adhesive further absorbs the water in the tissue fluid and blood; then, the absorbed water is gradually mixed with the dispersing agent, and gradually infiltrates the functional component and auxiliary crosslinking agent, so that the functional component and auxiliary crosslinking agent are fully dissolved, thereby triggering the adhesive molecular fragments in the functional component to react with the amino groups on the assistant crosslinker agent or the adhesive molecules themselves to cross-link. Herein, the medical tissue adhesive will be glued and solidified, and finally the wound can be sealed without bleeding.

### The function and effect of the invention

According to the medical tissue adhesive and the preparation method of the present invention, because of the functional components of the adhesive molecular fragments, auxiliary crosslinking agent and dispersing agent, the medical tissue adhesive provided by the present invention can not only use the dispersing agent to squeeze out the tissue fluid or blood on the tissue surface, so that the adhesive molecular fragments in the functional component can quickly form chemical bonds with the amino groups on the tissue surface to obtain strong tissue adhesion, and it can also trigger the functional components of the adhesive molecular fragments to react with the amino groups on the assistant crosslinker agent, so that the medical tissue adhesive is quickly glued and solidified, thereby granting it with good blood expelling performance, hemostatic performance, tissue adhesion performance and wound sealing performance.

### Description of figures

Figure 1 is a diagram of the medical tissue adhesive in embodiment 1;
Figure 2 is a diagram showing the efficacy of using the medical tissue adhesive for blood expelling in embodiment 2;
Figure 3 is a diagram showing the effect of using the medical tissue adhesive of embodiment 2 in the water absorption and gelation performance tests;
Figure 4 is a diagram showing the effect of using the medical tissue adhesive of embodiment 7 in the water absorption and gelation performance tests;
Figure 5 is a diagram of a rabbit liver hemorrhage model in the test case 4;
Figure 6 is a diagram of the using effect of the medical tissue adhesive in the test case 4;
Figure 7 is a diagram of a rabbit abdominal aortic hemorrhage model in the test case 2;
Figure 8 is a diagram showing the using effect of the medical tissue adhesive in the test case 2;
Figure 9 is a using effect diagram of the medical tissue adhesive in the test case 7;
Figure 10 is a diagram showing the using effect of the medical tissue adhesive in the test case 6;
Figure 11 is a diagram of the medical tissue adhesive in the embodiment 8 of the present invention;
Figure 12 is a diagram of the medical tissue adhesive in the embodiment 9 of the present invention;
Figure 13 is a diagram of the medical tissue adhesive in the embodiment 10 of the present invention;
Figure 14 is a diagram showing the effect of using the medical tissue adhesive of the present invention in the water absorption and gelation tests in the embodiment 11;
Figure 15 is another diagram showing the effect of using the medical tissue adhesive of the present invention in the water absorption and gelation tests in the embodiment 8;
Figure 16 is a diagram of the water absorption and gelation performance tests of the medical tissue adhesive in the test case 4 of the present invention;
Figure 17 is a diagram of the water absorption, gelation performance and swelling tests of the medical tissue adhesive in the test case 4 of the present invention;
Figure 18 is a diagram of the water absorption and gelation performance tests of the medical tissue adhesive in the test case 5 of the present invention;
Figure 19 is a diagram of the initial effect of the medical tissue adhesive in the test case 6 of the present invention;
Figure 20 is a diagram of the after-sealing effect of the medical tissue adhesive in the test case 6 of the present invention;
Figure 21 is a diagram of rabbit abdominal aortic hemorrhage in the test case 7 of the present invention;
Figure 22 is a diagram of rabbit abdominal aorta after hemostasis in the test case 7 of the present invention;
Figure 23 is a diagram of rabbit femoral artery hemorrhage in the test case 7 of the present invention;
Figure 24 is a diagram of ordinary medical gauze used in the test case 7 as a control group of the present invention;
Figure 25 is a diagram showing the effect of using ordinary medical gauze in the test case 7 of the present invention;
Figure 26 is a diagram showing the effect of using medical tissue adhesive in the test case 7 of the present invention;
Figure 27 is a diagram for the effect of the medical tissue adhesive in the test case 7 of the present invention after use;
Fig. 28 is a diagram of the adhesive strength test of the medical tissue adhesive in the test case 8 of the present invention.

### Mode of carrying out the invention

The following is a detailed description of the medical tissue adhesives prepared by liquid dispersing agents or solid dispersing agents.

In order to make it easy to understand the technical means, creative features, objectives and effects of the present invention, a medical tissue adhesive and its preparation method, will be described in detail below with reference to the embodiments and figures.

In the following embodiments, in order to simplify the description, the N-hydroxysuccinimide is replaced by its abbreviation "NHS"; the o-nitrobenzyl light trigger is replaced by its abbreviation "NB"; 1-(3-dimethyl Aminopropyl)-3-ethylcarbodiimide hydrochloride is replaced by its abbreviation "EDC", 4-(4,6-dimethoxytriazin-2-yl)-4-methylmorpholinate is replaced by its abbreviation "DMTMM".

The molecular weight distributions of some of the raw materials used in the following embodiments are as follows:
Polyglutamic acid: molecular weight is o.7 million to 1 million;
Polyethyleneimine: molecular weight is 70,000;
Hyaluronic acid: molecular weight is 1 million;
Polylysine: molecular weight is 1500;
Dextran: molecular weight is 100,000;
Carboxyl-terminated four-arm polyethylene glycol: molecular weight is 2000;
Amino-terminated four-arm polyethylene glycol: molecular weight is 2000.

In the following embodiments 1 to 7 and test cases 1 to 3, the medical tissue adhesive is prepared by liquid dispersing agent.

### <Embodiment 1>

The medical tissue adhesive provided in this embodiment is prepared by parts by weight of 1 part of NB modified polyglutamic acid with a grafting rate of 10%, 0.01 part of polyethyleneimine, and 1 part of 1,2-propylene glycol.

Specifically, the preparation method of NB modified polyglutamic acid (PGNAB) is to dissolve aminated NB, polyglutamic acid, EDC and NHS into water at a mass ratio of 1:15:1:1, adjust the pH to 5.5, heat to 35°C, and stir for 3 hours. After dialysis and lyophilization, the NB-modified polyglutamic acid with a grafting rate of 10% is obtained.

The structural formula of aminated NB is shown below:

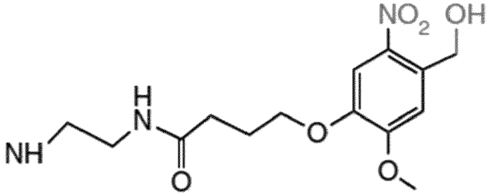

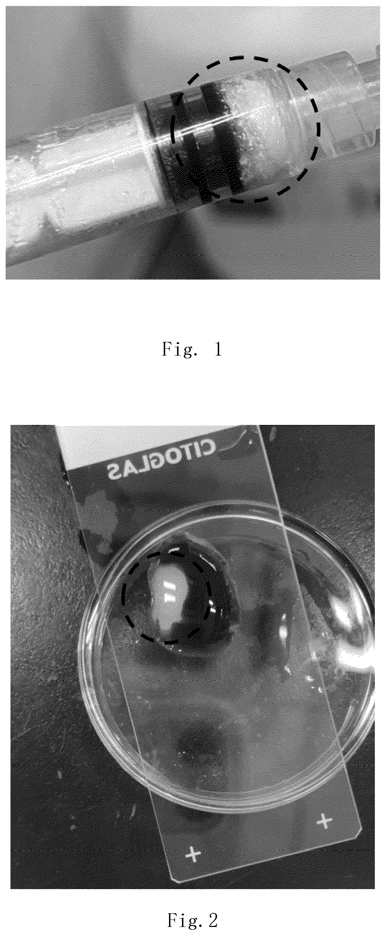

The preparation method of the medical tissue adhesive provided in this embodiment is: NB modified polyglutamic acid (with a grafting rate of 10%), polyethyleneimine, and 1,2-propanediol are mixed uniformly in a mass ratio of 1:0.01:1. The final product is shown in Figure 1.

### <Embodiment 2>

The medical tissue adhesive provided in this embodiment is prepared from 1 part by weight of NHS-modified hyaluronic acid (HANHS) with a grafting rate of 10%, 0.5 part by weight of polylysine, and 2 parts by weight of glycerin.

Specifically, the preparation method of NHS-modified hyaluronic acid (HANHS) is to dissolve hyaluronic acid, EDC and NHS in water at a mass ratio of 10:1:1, adjust the pH to 5.5, heat to 35°C, and stir for 1 hour. After dialysis and lyophilization, NHS-modified hyaluronic acid with a grafting rate of 10% was obtained.

The preparation method of the medical tissue adhesive provided in this embodiment is as follows: NHS-modified hyaluronic acid with a grafting rate of 10%, polylysine and glycerin with a grafting rate of 10% are mixed uniformly in a mass ratio of 1:0.5:2.

### <Embodiment 3>

The medical tissue adhesive provided in this embodiment is prepared from 1 part by weight of aldehyde-modified dextran (ODEX) with a 30% aldehyde grafting rate, 0.2 part by weight of gelatin, and 1.5 parts by weight of ethylene glycol.

Specifically, the preparation method of aldehyde modified dextran is to dissolving dextran and sodium periodate in water at a molar ratio of 1:1, adjust the pH to 4.5, and stir for 1.5 hours. After dialysis and lyophilization, the aldehyde modified dextran with a grafting rate of 30% was obtained.

The preparation method of the medical tissue adhesive provided in this embodiment is as follows: aldehyde group-modified dextran with a grafting rate of 30% aldehyde group, gelatin and ethylene glycol are mixed uniformly in a mass ratio of 1:0.1:30.

### <Embodiment 4>

The medical tissue adhesive provided in this embodiment is composed of 1 part by weight of four-arm polyethylene glycol modified with an active ester (4a-PEG-NHS) with a grafting rate of 100%, and 0.2 part of amino-terminated four-arm polyethylene glycol (4a-PEG-NH₂) and 1.5 parts of glycerin.

Specifically, the preparation method of amino-terminated four-arm polyethylene glycol is to dissolve carboxyl-terminated four-arm polyethylene glycol (4a-PEG-COOH), EDC and NHS into water at a mass ratio of 1:0.1:0.1, adjust the pH to 5.5, heat to 35° C, stir for 0.5 hours, followed by dialysis and lyophilization to obtain the active ester modified four-arm polyethylene glycol with a grafting rate of 100%.

The preparation method of the medical tissue adhesive provided in this embodiment is as follows: NHS-modified hyaluronic acid with a grafting rate of 10%, polylysine and glycerin with a grafting rate of 10% are mixed uniformly in a mass ratio of 1:0.5:2.

### <Embodiment 5>

The medical tissue adhesive provided in this embodiment is composed of 1 part by weight of microparticles modified by tissue adhesive groups (mHA-NHS, microsphere diameter of 5 micrometers) with a grafting rate of 100%, 0.5 part of chitosan and 2 parts of glycerin. Specifically, the preparation method of microparticles modified with tissue adhesive groups is to dissolve hyaluronic acid, EDC amd NHS in water at a mass ratio of 1:0.1:0.1, adjust the pH to 5.5, heat to 35°C, and stir for 0.5 hours. Followed by centrifugation and washing 3 times, microparticles (mHA-NHS) modified with tissue adhesive groups with a grafting rate of 100% was obtained.

The preparation method of the medical tissue adhesive provided in this embodiment is as follows: the microparticles modified by tissue adhesive groups with a grafting rate of 100%, chitosan and glycerin are mixed uniformly in a mass ratio of 1:0.5:2.

### <Embodiment 6>

The medical tissue adhesive provided in this embodiment is composed of 1 part by weight of NB-modified hyaluronic acid (HANB) with a grafting rate of 4%, 0.5 part by weight of Chitosan nanoparticles (nCS, 50 nanometers in diameter) and 1.5 parts of glycerol.

Specifically, the preparation method of the NB-modified hyaluronic acid is to dissolve NB, hyaluronic acid, EDC and NHS into water at a mass ratio of 1:20:1:1, adjust the pH to 5.5, heat to 35°C, stir for 2 hours. After dialysis and lyophilization, the NB-modified hyaluronic acid with a grafting rate of 4% is obtained.

The preparation method of the medical tissue adhesive provided in this embodiment is as follows: the NB-modified hyaluronic acid with a grafting rate of 4%, chitosan nanoparticles and glycerin are mixed uniformly in a mass ratio of 1:0.5:1.5.

### <Embodiment 7>

The medical tissue adhesive provided in this embodiment is prepared from 1 part by weight of NHS-modified hyaluronic acid (HANHS) with a grafting rate of 10%, 0.5 part of polylysine, and 2 parts of silicone oil.

Specifically, the preparation method of NHS-modified hyaluronic acid (HANHS) is the same as in embodiment 2.

The preparation method of the medical tissue adhesive provided in this embodiment is: NHS-modified hyaluronic acid with a grafting rate of 10%, polylysine and silicone oil are mixed uniformly in a mass ratio of 1:0.5:2.

### <Test case 1>

The test of blood resistant performance and water absorption and gel forming performance The method for testing the blood resistant performance is as follows: 2ml of blood was dripped on a glass slide, 2ml of the medical tissue adhesive provided in Embodiment 2 or Embodiment 7 was then dripped on the blood.

The results of the blood resistant performance are shown in Figure 2.

As shown in Figure 2, the medical tissue adhesive in Embodiment 2 can quickly drain the blood and contact with the bottom of the glass slide, then absorb the water in blood to solidify into a gel owing to the use of the hydrophilic dispersing agent, glycerin. These results prove the excellent performance of this medical tissue adhesive in blood resistance, water-absorbing and gel-forming. Since the medical tissue adhesive in Embodiment 7 also utilizes the hydrophobic dispersing agent, silicone oil, it can drain blood rapidly and contact with the bottom of the glass slide.

The test method of water absorption and gel forming performance is as follows: 5 ml of deionized water was added to the petri dish, and then 0.2 ml of the medical tissue adhesive in Embodiment 2 or Embodiment 7 was dripped into the water.

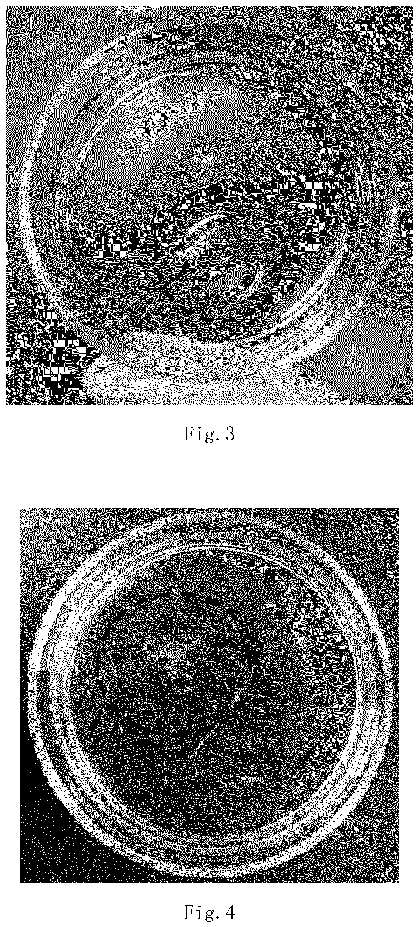

The results of water absorption and gel forming performance are shown in Figure 3-4.

As shown in Figure 3, since the hydrophilic dispersing agent glycerin used in the medical tissue adhesive of Embodiment 2 is miscible with water, it will quickly absorb water and dissolve the functional component and auxiliary crosslinking agent to form a gel. And the color gradually changes from milky white to transparent from outside to inside. It will completely become transparent after 2 minutes, forming a complete gel.

However, as shown in Figure 4, the medical tissue adhesive in Embodiment 7 uses silicone oil, a hydrophobic dispersing agent that is not miscible with water, which will trap the functional component and auxiliary crosslinking agent, making it difficult for water to contact and dissolve them. As the soaking time increases, the latex gel will gradually be dispersed by water from outside to inside to form milky white granules, which will eventually lead to the inability to form a complete mass of gel.

### <Test case 2>

The test of tissue adhesion performance, wound sealing performance and hemostatic performance

The first test method is as follows: Firstly, as shown in Figure 4, a liver injury model in rabbits was created by using a 2mm diameter needle, and a large amount of blood flow can be observed; then, the medical tissue adhesive in Embodiment 4 was squeezed onto the gauze immediately and pressed to the wound for 10 seconds, and then the gauze was removed.

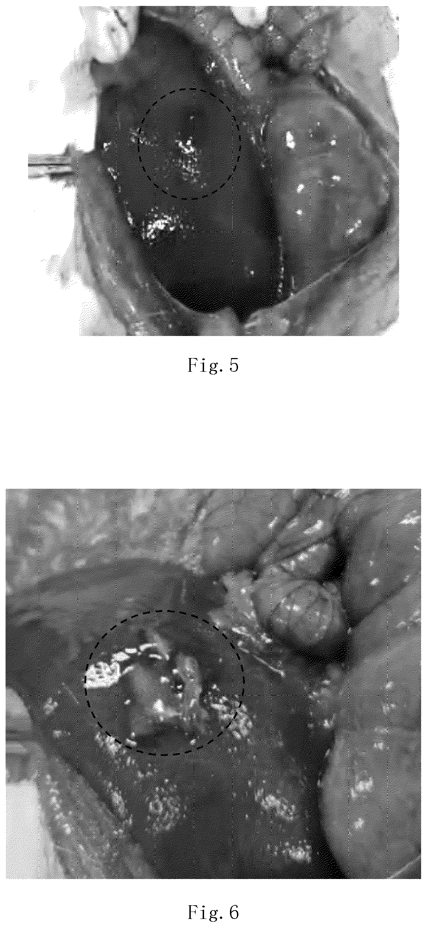

The test result is shown in Figure 5.

As shown in Figure 5, the medical tissue adhesive can firmly adhere to the liver surface and form a gel by absorbing the water in blood, and no blood flows was out at the same time, which proves the good tissue adhesion performance, wound sealing performance and hemostatic performance of this tissue adhesive.

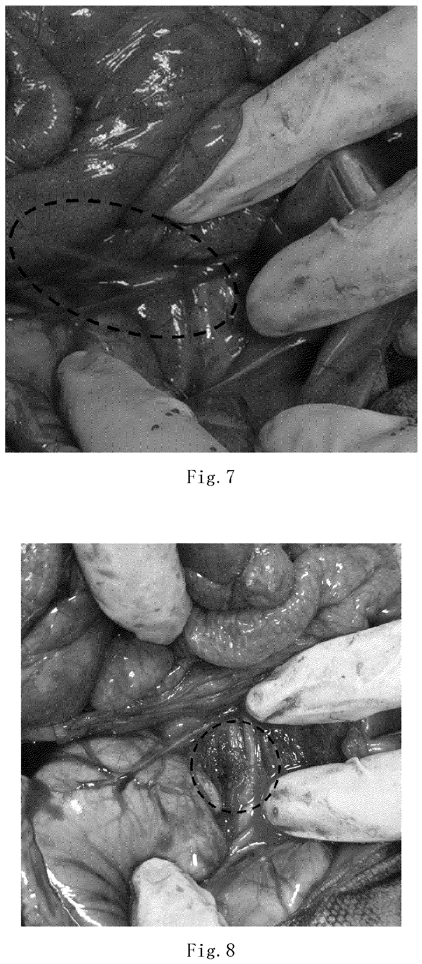

The second test method is as follows: Firstly, as shown in Figure 7, a 2mm diameter needle was used to create a massive hemorrhage model of the abdominal aorta injury in rabbits, and a large amount of blood column can be observed; Then, the medical tissue adhesive in Embodiment 2 or Embodiment 7 was squeezed onto the gauze immediately and pressed to the wound for 10 seconds, and then the gauze was removed.

The test results are shown in Figure 8-9.

As shown in Figure 8, the medical tissue adhesive in Embodiment 2 can strongly adhere to the surface of the abdominal aorta and has absorbed the water in blood to form a gel. At the same time, there was no more blood flow and the abdominal aorta and the gel adhering to the defect can be clearly observed. Above results proved the good tissue adhesion performance, wound sealing performance and hemostatic performance of this tissue adhesive.

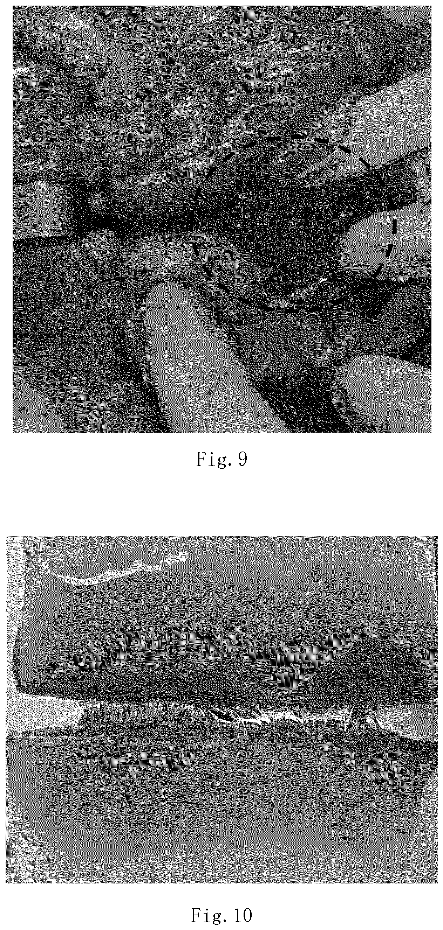

However, as shown in Figure 9, the medical tissue adhesive provided in Embodiment 7 could not stop the severe bleeding from abdominal aortic injury, and there was still a large amount of blood ejected rapidly, and a large amount of blood flooded the abdominal cavity, which proved that the use of hydrophobic dispersing agent in this medical tissue adhesive cannot seal the wound and stop bleeding effectively.

### <Test case 3>

### Adhesion rate test

The test method is as follows: The medical tissue adhesive in Embodiments 1 to 7 was applied between two pieces of pigskin, with a smearing amount of 0.1 mL/cm², and then the pigskin coated with the medical tissue adhesive was soaked in water, and the adhesion time was recorded.

The test results are shown in Table 1 and Figure 10.

**Table 1 The results of adhesion rate test**

| Serial number | Embodiment 1 | Embodiment 2 | Embodiment 3 | Embodiment 4 | Embodiment 5 | Embodiment 6 | Embodiment 7 |
|---|---|---|---|---|---|---|---|
| Adhesion time (s) | <60s | < 10s | < 20s | < 20s | < 30s | <60s | Unable to adhere |

As shown in Figure 10, the tissue adhesives provided in Embodiment 1 to 6 can form a gel and be solidified within 60 seconds, and adhere two pieces of pigskin together. The adhesion rate is very high, and it can be widely used under various medical conditions.

In the following Embodiments 8 to 17 and Test case 4 to 8, the medical tissue adhesives used were prepared by the solid dispersing agent.

### <Embodiment 8>

The medical tissue adhesive provided in this Embodiment is composed of 1 part by weight of four-arm polyethylene glycol modified with active ester (4a-PEG-NHS) with a grafting rate of 100%, 0.12 parts of polyethyleneimine (PEI) and 3 parts of polyethylene glycol-1000 (PEG-1000).

Among them, the preparation of four-arm polyethylene glycol modified with active ester (4a-PEG-NHS) is as follows: The carboxyl-terminated four-arm polyethylene glycol (4a-PEG-COOH), EDC, and NHS at the mass ratio of 1 :0.1: 0.1 were dissolved in water, adjusted pH to 5.5, heated to 35°C, stirred for 0.5 hours, then dialyzed and lyophilized to obtain four-arm polyethylene glycol modified with active ester with a grafting rate of 100%.

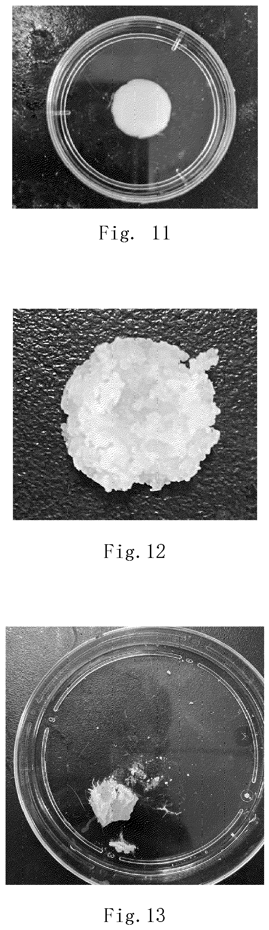

The preparation of the medical tissue adhesive provided in this Embodiment is as follows: Four-arm polyethylene glycol modified with active ester with a grafting rate of 100%, amino-terminated four-arm polyethylene glycol, and polyethylene glycol-1000 in accordance with the mass ratio of 1: 0.12: 3 were prepared. After polyethylene glycol-1000 was melted under dry heating conditions at 50°C, four-arm polyethylene glycol modified with active ester with a grafting rate of 100% and amino-terminated four-arm polyethylene glycol were added and mixed well, and then cooled down in molds. The final product is shown in Figure 11.

### <Embodiment 9>

The medical tissue adhesive provided in this Embodiment is prepared from 1 part by weight of microparticles modified with tissue adhesive groups (mHA-NHS, with the diameter of 5 µm) with a grafting rate of 100%, 0.5 parts of chitosan and 2 parts of polyethylene glycol 1000. Among them, the preparation method of microparticles modified with tissue adhesive groups is as follows: Hyaluronic acid, EDC and NHS were dissolved in water at a mass ratio of 1: 0.1: 0.1; the pH of the solution was adjusted to 5.5, then the solution was heated to 35°C, stirred to react for 0.5 hours, followed by centrifugation and washed 3 times and microparticles (mHA-NHS) modified with tissue adhesive groups with a grafting rate of 100% were obtained. The preparation of the medical tissue adhesive provided in this Embodiment is as follows: After the polyethylene glycol 1000 was melted at 50°C, the polyethylene glycol 1000, microparticles modified with the tissue adhesive group with a grafting rate of 100% and chitosan at the mass ratio of 2:1:0.5 were mixed uniformly to obtain the final medical tissue adhesive. The final product is shown in Figure 12.

### <Embodiment 10>

The medical tissue adhesive provided in this embodiment is composed of 1 part of acrylate-terminated four-arm polyethylene glycol (4a-PEG-AA), 1 part of ammonium persulfate, 1 part of ferrous chloride, and 2 parts of polyethylene glycol 1000 (PEG-1000), based on mole parts. The preparation method of the medical tissue adhesive provided in this embodiment is as follows: Acrylate-terminated four-arm polyethylene glycol, ammonium persulfate, ferrous chloride, and polyethylene glycol 1000 in a molar ratio of 1:1: 1: 2 were mixed uniformly under the condition of 50°C dry heating, and the medical tissue adhesive was obtained by cooling down in molds. The final product is shown in Figure 13.

### <Embodiment 11>

The medical tissue adhesive provided in this embodiment is composed of 1 part by weight of polyglutamic acid modified with methacrylic anhydride (PGAMA) with a grafting rate of 10%, 0.1 part of sodium periodate, 0.1 part of vitamin C and 3 parts of polyethylene glycol 1500 (PEG-1500).

Among them, the preparation of methacrylic anhydride-modified polyglutamic acid (PGAMA) is as follows: 1 part of polyglutamic acid by weight was completely dissolved in deionized water, and the carboxyl content of polyglutamic acid and methacrylic anhydride at a molar ratio of 10: 1 were added to the polyglutamic acid solution. The pH of solution was adjusted to 8-9 and the reaction was stirred for 4 hours, dialyzed and lyophilized to obtain polyglutamic acid modified with methacrylic anhydride with a grafting rate of 10%.

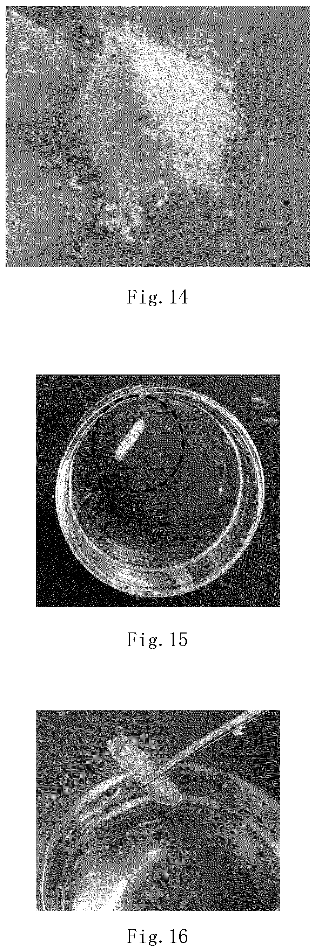

The preparation method of the medical tissue adhesive provided in this embodiment is as follows: Polyglutamic acid modified with methacrylic anhydride with a grafting rate of 10%, sodium periodate, vitamin C and polyethylene glycol 1500 at a mass ratio of 1: 0.1: 0.1: 3 were mixed uniformly under the condition of 50°C dry heating. After cooling, the sample is ground into powder. The final product is shown in Figure 14.

### < Embodiment 12>

The medical tissue adhesive provided in this embodiment is composed of 1 part by weight of four-arm polyethylene glycol modified with an active ester (4a-PEG-NHS) with a grafting rate of 100%, 0.2 parts of amino-terminated four-arm polyethylene glycol (4a-PEG-NH₂) and 1.5 parts of polyethylene glycol 1000 (PEG-1000).

Among them, the preparation method of the four-arm polyethylene glycol modified with an active ester (4a-PEG-NHS) is as follows: the carboxyl-terminated four-arm polyethylene glycol (4a-PEG-COOH), EDC and NHS at the mass ratio of 1: 0.1: 0.1 were dissolved in water, adjusted pH to 5.5, heated to 35°C, stirred for 0.5 hours, then dialyzed and lyophilized to obtain active ester modified four-arm polyethylene glycol with a grafting rate of 100%.

The preparation method of the medical tissue adhesive provided in this embodiment is as follows: Four-arm polyethylene glycol modified with active ester with a grafting rate of 100%, amino-terminated four-arm polyethylene glycol, and polyethylene glycol 1000 at the mass ratio of 1: 0.2: 1.5 were mixed uniformly under the condition of 50°C dry heating, and the medical tissue adhesive was obtained by cooling down in molds.

### < Embodiment 13>

The medical tissue adhesive provided in this embodiment is composed of 1 part of sodium periodate, 1 part of vitamin C and 2 parts of acrylate-terminated four-arm polyethylene glycol 1000 (4a-PEG- AA-1000), by mole ratio.

The preparation method of the medical tissue adhesive provided in this embodiment is as follows: sodium periodate, vitamin C and acrylate-terminated four-arm polyethylene glycol 1000 in a mass ratio of 1: 1: 2 were dried and heated at 50°C and the final adhesive was obtained by cooling down in molds.

### < Embodiment 14>

The medical tissue adhesive provided in this embodiment is composed of, by weight, 1 part a four-arm polyethylene glycol modified with an active ester (4a-PEG-NHS) with a grafting rate of 100%, 0.12 parts of polyethyleneimine (PEI) and 2 parts of maltose.

Among them, the preparation method of the four-arm polyethylene glycol modified with active ester is as follows: The carboxyl-terminated four-arm polyethylene glycol (4a-PEG-COOH), EDC and NHS were dissolved in water at the mass ratio of 1: 0.1: 0.1, adjusted the pH to 5.5, heated to 35°C, stirred and reacted for 0.5 hours, followed by dialysis and lyophilization to obtain the active ester modified four-arm polyethylene glycol with a grafting rate of 100%. The preparation method of the medical tissue adhesive provided in this embodiment is as follows: Maltose was melted under dry heating at 150°C, and mixed uniformly with four-arm polyethylene glycol modified with an active ester (4a-PEG-NHS) with a grafting rate of 100% and polyethylene (PEI) at the weight ratio of 2: 1: 0.12, and the final adhesive was obtained by cooling down in molds.

### < Embodiment 15>

The medical tissue adhesive provided in this embodiment, used as a comparative example, is composed of sodium periodate and acrylate-terminated four-arm polyethylene glycol 1000 (4a-PEG-AA-1000) at a mole ratio of 1:1.

The preparing method of the medical tissue adhesive provided in this embodiment is as follows: sodium periodate and acrylate-terminated four-arm polyethylene glycol 1000 are mixed uniformly at a mass ratio of 1:1 under dry condition at 50°C and then poured into the mold. The medical tissue adhesive is obtained when the mold is cooled.

### < Embodiment 16>

The medical tissue adhesive provided in this embodiment consists of 1 part of sodium periodate, 0.5 part of vitamin C and 1 part of acrylate-terminated four-arm polyethylene glycol 1000 (4a-PEG- AA-1000), by mole ratio.

The preparing method of the medical tissue adhesive provided in this embodiment is as follows: The acrylate-terminated four-arm polyethylene glycol 1000 was mixed with sodium periodate and vitamin C at a mass ratio of 1:1:0.5 under dry condition at 50°C and then poured into the mold. The medical tissue adhesive is obtained when the mold is cooled.

### < Embodiment 17>

The medical tissue adhesive provided in this embodiment is prepared by 1 part of vitamin C and 1 part of acrylate-terminated four-arm polyethylene glycol 1000 (4a-PEG-AA-1000), by mole ratio.

The preparing method of the medical tissue adhesive provided in this embodiment is as follows: vitamin C and acrylate-terminated four-arm polyethylene glycol 1000 are mixed uniformly at a mass ratio of 1:1 under dry conditions at 50°C and then poured into the mold. The medical tissue adhesive is obtained when the mold is cooled.

### <Test case 4>

### Water absorption and gel forming performance

The first test method of water absorption and gelling performance is as follows: add 2ml of deionized water in a petri dish, and then place a 10mm*1mm cylindrical medical tissue adhesive provided in Embodiment 8 into the water.

The test results of water absorption and gelling performance are shown in Figure 15 and Figure 16. As shown in Figure 15, the medical tissue adhesive provided in Embodiment 8 possesses the solid dispersing agent polyethylene glycol, which induce to drain liquids such as water by gravity and contact the bottom of the petri dish, absorbing water to solidify into a glue. It proves that the medical tissue adhesive owns properties of good drainage, water absorption and gel forming performance.

As shown in Figure 16, the hydrophilic dispersing agent polyethylene glycol used in the medical tissue adhesive provided in Embodiment 8 can be dissolved in water, which can be quickly dissolved to form a gel with the functional component and auxiliary crosslinking agent dissolved in water. The color gradually changes from milky white to colorless and transparent from the outside to the inside. After 5 minutes, it becomes colorless and transparent, forming a complete glue.

The second test method for water absorption and gelation performance is as follows: add 2 ml of deionized water into a petri dish, and then place the medical tissue adhesive provided in Embodiment 9 with a disc shape of 10 mm in diameter into the water.

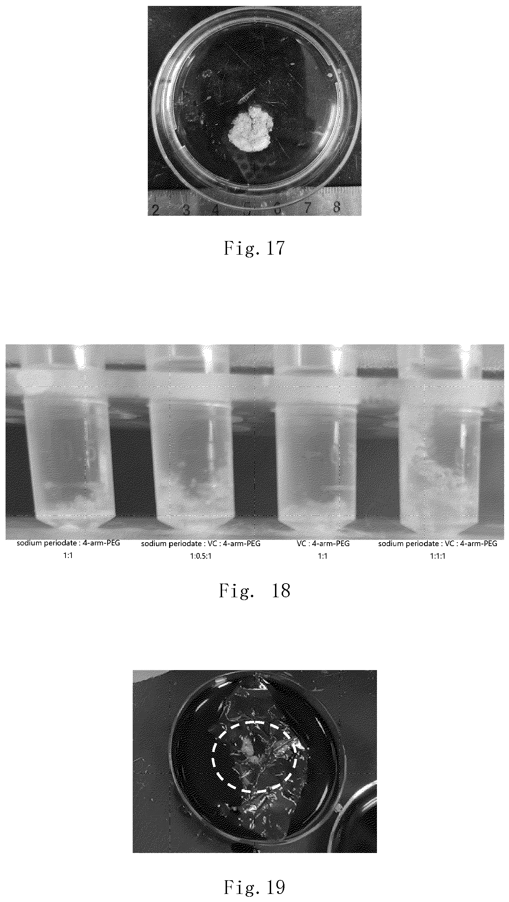

Figure 17 shows the test results of water absorption and gel forming performance.

As shown in Figure 17, the medical adhesive provided in Embodiment 9 has the hydrophilic solid dispersing agent polyethylene glycol, which can react with the auxiliary crosslinking agent to form glue during the process of dissolution in water. Meanwhile, the adhesive glue changes from white to colorless and transparent, forming a complete glue in the form of interconnected micron particles, and hardly swells.

### <Test case 5>

### Water absorption and gel forming performance test

The method of water absorption performance is as follows: add 1 mL of deionized water to the centrifuge tube, then add 100 mg of medical tissue adhesives provided in the Embodiment 13, Embodiment 15, Embodiment 16, and Embodiment 17 (weight as four-arm polyethylene glycol 1000) into the water.

Figure 18 shows the results of the water absorption and gel forming performance.

As shown in Figure 18, the medical tissue adhesive provided in Embodiment 13 can form a complete glue due to the auxiliary crosslinking agent with redox properties and the four-arm polyethylene glycol as the functional component of the solid dispersing agent. Since the content of the auxiliary crosslinking agent is not optimal, only part of the hydrogel can be formed in Embodiment 16. However, since Embodiment 15 and Embodiment 17 only contain a single oxidizing agent or reducing agent, they cannot produce the effect of auxiliary cross-linking, so they cannot be gelled when dispersed in water. It proves the necessity of assistant crosslinker agent in the formation of hydrogel.

### <Test case 6>

### Adhesion performance test in blood

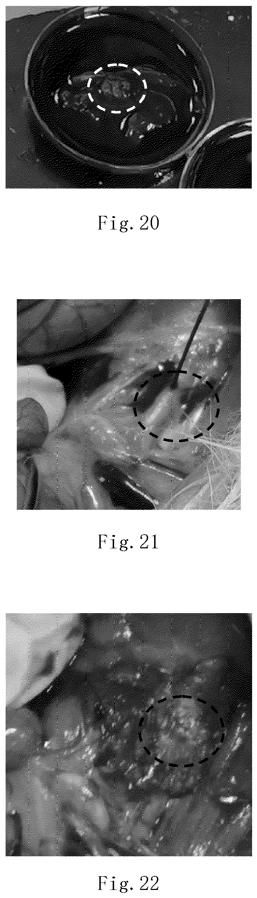

The test method is as follows: First, immerse the fresh tissue in the blood. Second, take 200 mg of the medical tissue adhesive in Embodiment 14 and press it on the surface of bloody tissue for 15 seconds as shown in Figure 19, and then let it lay in the blood for 3 minutes. The test result is shown in Figure 20. As shown in Figure 20, the medical tissue adhesive provided in Embodiment 14 can firmly adhere to the bloody tissue surface and form a gel, and the gel on the tissue can be clearly seen, which proves the adhesion performance of the medical tissue adhesive in the blood.

### <Test case 7>

Tissue adhesion performance, wound sealing performance and hemostatic performance tests

Test method one: first, as shown in Figure 21, a 2mm diameter needle is used to create a massive hemorrhage model of abdominal aortic injury on the rabbit's abdominal aorta, and a large amount of blood column can be observed; the medical tissue adhesive of Embodiment 8 is pressed to the injury for 15 seconds, and then released.

The test result is shown in Figure 22.

As shown in Figure 22, the medical tissue adhesive provided in the Embodiment 8 can firmly adhere to the surface of the abdominal aorta and has crosslinked by absorbing water in the blood. At the same time, there is no more blood flowing out; the abdominal aorta can be clearly seen as well as the gel adhering to the defect, which proved the good tissue adhesion performance, wound sealing performance and hemostatic performance of the medical tissue adhesive.

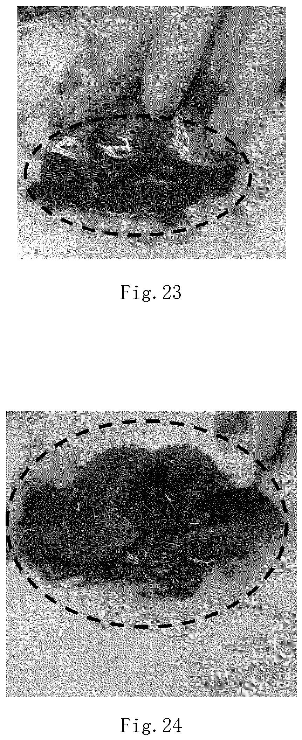

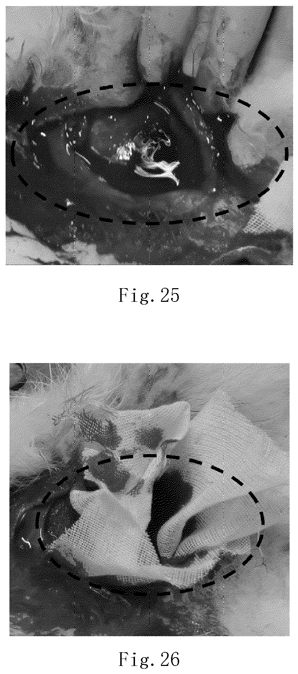

Test method two: first, as shown in Figure 23, the rabbit femoral artery was directly cut with a scalpel, and a large amount of blood gushing can be observed; then, ordinary medical gauze was immediately pressed to the wound bleeding site, and it can be seen that the gauze was quickly stained red with blood. After 3 minutes of pressing, there was still a lot of blood seeping through the gauze, as shown in Figure 24. After the gauze was removed, a large amount of blood was still gushing out, and the bleeding was not reduced, as shown in Figure 25.

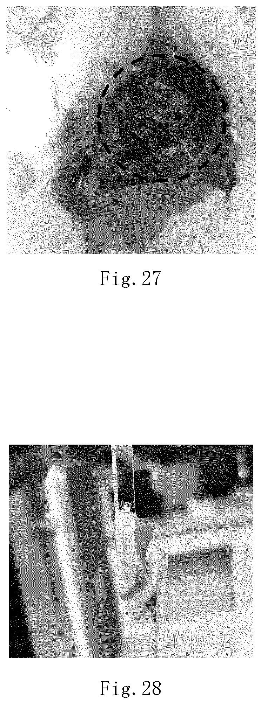

The test results are shown in Figure 26 and Figure 27.

As shown in Figure 26, in the case of severe hemorrhage with the same femoral artery cut, the medical tissue adhesive provided in the Embodiment 10 was spread on the gauze and pressed to the injury for 10 seconds, and then the pressure was released. It can be seen that the gauze was not continuously infiltrated by blood, which suggested that bleeding was stopped. After another 3 minutes, no blood continued to stain the gauze and no blood continued to ooze out, which proves that the medical tissue adhesive provided in the embodiment 10 has performance, wound sealing performance and hemostatic performance.

As shown in Figure 27, after the gauze was removed, it could be seen that the wound was covered and sealed by a layer of gel, and no blood continued to flow out, which proves that the medical tissue adhesive has strong sealing and hemostasis capabilities.

### <Test case 8>

### Adhesive strength test

The test method is as follows: sandwich the medical tissue adhesive of Embodiment 12 between two pieces of pigskin, the usage amount is 0.1cm*0.1cm*0.01cm, and then soak the pigskin coated with tissue adhesive in water. After 5 minutes, pull the two pigskins with a universal testing machine.

The test result is shown in Figure 28.

As shown in Figure 28, the medical tissue adhesive of the Embodiment 12 could bond two pieces of pigskin together. After pulling, there was still gel remaining between the two pieces of pigskin, which proves that the medical tissue has strong adhesive strength.

### The function and effect of the embodiments

According to the medical tissue adhesive and the preparation method of Embodiment 1 to 6, the dispersing agent used in these embodiments is a liquid dispersing agent which will squeeze the blood away when it comes in contact with the blood. So the adhesive group on the functional components can fully contact and react with the amino groups on the tissue surface, without being affected by the amino group reaction on the protein in the blood and tissue fluid. And then, the hydrophilic liquid dispersing agent will absorb and be dissolved in the water in the blood and tissue fluid, and the functional components will be dissolved and the adhesive molecular fragments in them will be triggered and react with the amino groups on the assistant crosslinker agent, making the whole medical tissue adhesive quickly gelled and solidified. Therefore, the medical tissue adhesive prepared in Embodiments 1 to 6 can not only have good bleed resistant performance, but also can be quickly cross-linked to form a gel to achieve rapid adhesion of tissues and seal wounds.

According to the medical tissue adhesive and the preparation method in Embodiment 8 to 17, the dispersing agent used in these embodiments is a solid hydrophilic dispersing agent, and the solid dispersing agent will be in contact with the surface of tissue due to gravity and density when it is contact with the blood. As it absorbs and dissolves in the water, the adhesive groups on the functional component can fully contact and react with the amino groups on the tissue surface, and will not be affected by the amino groups on the protein in the blood and tissue fluid. Afterwards, the solid hydrophilic dispersing agent will absorb water in blood and tissue fluid, further dissolve the functional components, trigger the adhesive molecular fragments in the functional component to react with the amino group on the assistant crosslinker agent, and make the whole medical tissue adhesive quickly gelled and solidified. Therefore, the medical tissue adhesives prepared in Embodiment 8 to 17 can also be quickly cross-linked to form a gel to achieve rapid adhesion of tissues and seal wounds.

The foregoing embodiments are preferred cases of the present invention, and are not used to limit the protection scope of the present invention.

## Claims

1. A medical tissue adhesive for adhering biological tissue comprising:
a functional component, comprising polymer chains or micro/nano particles modified with tissue-adhering functional groups;
an assistant crosslinker, comprising chemicals that crosslink or stimulate crosslinking of the tissue-adhering functional groups; and
a dispersing agent, comprising a compound which can facilitate contact between the functional components and the biological tissue upon triggered by water-containing fluid.

2. The medical tissue adhesive according to claim 1, **characterized in that**:
the tissue-adhering functional group comprising: o-Nitrobenzyl photo-trigger group, active ester group, isocyanate group, isothiocyanate group, epoxide group, cyclic carbonate group, cyclic thiocarbonate group, active carbonyl group, or active double bond group,
wherein the o-Nitrobenzyl photo-trigger group is expressed by structural formula (I) and structural formula (II) below: 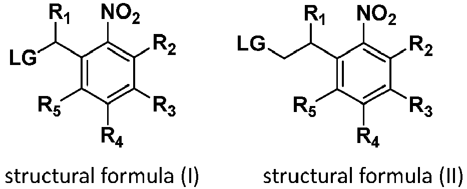
in formula (I) and (II), LG is selected from halogen atom, O-R', S-R', or NH-R'; wherein R' is one of the following groups: hydrogen, alkyl group, ether group, thioether group, carbonyl group, ester group, thioester group, amide group, sulfate group, sulphonate group, or phosphonate group;
R1 is one of the following groups: hydrogen, halogen atom, hydroxy group, mercapto group, amino group, nitro group, cyano group, aldehyde group, carbonyl group, ester group, amide group, thioester group, sulfate group, sulphonate group, phosphonate group, sulfuryl group, sulfoxide group, aryl group, alkyl group, or modified alkyl group,
one or more of R2, R3, R4, R5 are linked with the polymer chains or the micro/nano particles,
R2, R3, R4 or R5before linked with the polymer chains or the micro/nano particles is one of the following groups: hydrogen, halogen atom, hydroxy group, mercapto group, amino group, nitro group, cyano group, aldehyde group, carbonyl group, carboxyl group, ester group, thioester group, isocyanate group, isothiocyanate group, epoxide group, carbonate group, thiocarbonate group, cyclic carbonate group, cyclic thiocarbonate group, amide group, sulfate group, sulphonate group, phosphonate group, sulfuryl group, sulfoxide group, aryl group, alkyl group, or modified alkyl group,
the active ester group is a succinimide active ester group expressed by structural formula (III) or a triazole active ester group expressed by structural formula (IV) below: 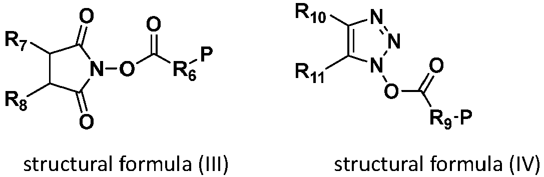
the succinimide active ester group is linked via R6 with the polymer chains or the micro/nano particles denoted as P,
in structural formula (III), R6 is directly linked with the polymer chains or the micro/nano particles, or indirectly linked with the polymer chains or the micro/nano particles via one of the following modified end groups: ether bond, thioether bond, carbonyl group, ester group, thioester group, amide group, sulfate group, sulphonate group, phosphonate group, sulfuryl group, sulfoxide group, aryl group, alkyl group, or modified alkyl group,
R7, R8 are either cyclized with each other or each selected from hydrogen, halogen atom, hydroxy group, mercapto group, amino group, nitro group, cyano group, aldehyde group, carbonyl group, ester group, thioester group, isocyanate group, isothiocyanate group, epoxide group, carbonate group, thiocarbonate group, cyclic carbonate group, cyclic thiocarbonate group, amide group, sulfate group, sulphonate group, phosphonate group, sulfuryl group, sulfoxide group, aryl group, alkyl group, or modified alkyl group,
the thiazole active ester group is linked via R9 with the polymer chains or the micro/nano particles denoted as P,
in structural formula (IV), R9 is directly linked with the polymer chains or the micro/nano particles, or indirectly linked with the polymer chains or the micro/nano particles via one of the following modified end groups: ether bond, thioether bond, carbonyl group, ester group, thioester group, amide group, sulfate group, sulphonate group, phosphonate group, sulfuryl group, sulfoxide group, aryl group, alkyl group, or modified alkyl group,
R10, R11 are either cyclized with each other or each selected from hydrogen, halogen atom, hydroxy group, mercapto group, amino group, nitro group, cyano group, aldehyde group, carbonyl group, ester group, thioester group, isocyanate group, isothiocyanate group, epoxide group, carbonate group, thiocarbonate group, cyclic carbonate group, cyclic thiocarbonate group, amide group, sulfate group, sulphonate group, phosphonate group, sulfuryl group, sulfoxide group, aryl group, alkyl group, or modified alkyl group,
the isocyanate group is expressed by structural formula (V) below:
**O=C=N-R₁₂** structural formula (V)
in structural formula (V), R12 is linked with the polymer chains or the micro/nano particles, and R12 before linked is one of the following end groups: end amino group, hydroxy group, mercapto group, halogen atom, acyl halide group, acid anhydride group, carboxyl group, carboxylate group, active ester group, isocyanate group, isothiocyanate group, epoxide group, cyclic carbonate group, cyclic thiocarbonate group, carbonate group, aryl group modified with active double bond group or active carbonyl group, heteroaromatic group, alkyl group, or modified alkyl group,
the isothiocyanate group is expressed by structural formula (VI) below:
**S=C=N-R₁₃** structural formula (VI)
in structural formula (VI), R13 is further linked with the polymer chains or the micro/nano particles, and R13 before linked is one of the following end groups: end amino group, hydroxy group, mercapto group, halogen atom, acyl halide group, acid anhydride group, carboxyl group, carboxylate group, active ester group, isocyanate group, isothiocyanate group, epoxide group, cyclic carbonate group, cyclic thiocarbonate group, carbonate group, aryl group modified with active double bond group or active carbonyl group, heteroaromatic group, alkyl group, or modified alkyl group,
the epoxide group is expressed by structural formula (VII) below: 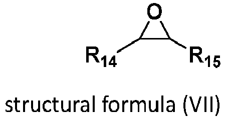
in structural formula (VII), R14 that is further linked with the polymer chains or the micro/nano particles, and R14 before linked is one of the following end groups: end amino group, hydroxy group, mercapto group, halogen atom, acyl halide group, acid anhydride group, carboxyl group, carboxylate group, active ester group, isocyanate group, isothiocyanate group, epoxide group, cyclic carbonate group, cyclic thiocarbonate group, carbonate group, aryl group modified with active double bond group or active carbonyl group, heteroaromatic group, alkyl group, or modified alkyl group,
in structural formula (VII), R15 is either cyclized with R14 or selected from hydrogen, halogen atom, hydroxy group, mercapto group, amino group, nitro group, cyano group, aldehyde group, carbonyl group, carboxyl group, ester group, thioester group, isocyanate group, isothiocyanate group, epoxide group, cyclic carbonate group, cyclic thiocarbonate group, carbonate group, thiocarbonate group, amide group, sulfate group, sulphonate group, phosphonate group, sulfuryl group, sulfoxide group, aryl group, alkyl group, or modified alkyl group,
the cyclic carbonate group is expressed by structural formula (VIII) below: 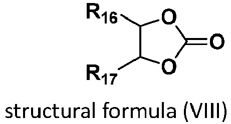
in structural formula (VIII), R16 is further linked with the polymer chains or the micro/nano particles, and R16 before linked is one of the following end groups: end amino group, hydroxy group, mercapto group, halogen atom, acyl halide group, acid anhydride group, carboxyl group, carboxylate group, active ester group, isocyanate group, isothiocyanate group, epoxide group, cyclic carbonate group, cyclic thiocarbonate group, carbonate group, aryl group modified with active double bond group or active carbonyl group, heteroaromatic group, alkyl group, or modified alkyl group,
in structural formula (VIII), R17 is either cyclized with R16 or selected from hydrogen, halogen atom, hydroxy group, mercapto group, amino group, nitro group, cyano group, aldehyde group, carbonyl group, carboxyl group, ester group, thioester group, isocyanate group, isothiocyanate group, epoxide group, cyclic carbonate group, cyclic thiocarbonate group, carbonate group, thiocarbonate group, amide group, sulfate group, sulphonate group, phosphonate group, sulfuryl group, sulfoxide group, aryl group, alkyl group, or modified alkyl group,
the cyclic thiocarbonate group is one of the seven chemicals expressed by structural formula (IX) below: 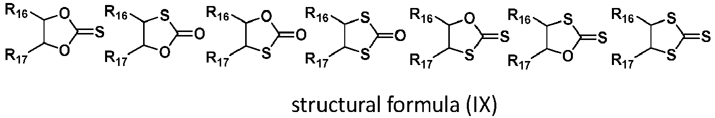
the active carbonyl group is expressed by structural formula (X) below: 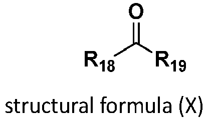
in structural formula (X), R18 is further linked with the polymer chains or the micro/nano particles, and R18 before linked is one of the following end groups: end amino group, hydroxy group, mercapto group, halogen atom, acyl halide group, acid anhydride group, carboxyl group, carboxylate group, active ester group, isocyanate group, isothiocyanate group, epoxide group, cyclic carbonate group, cyclic thiocarbonate group, carbonate group, aryl group modified with active double bond group or active carbonyl group, heteroaromatic group, alkyl group, or modified alkyl group,
in structural formula (X), R19 is either cyclized with R18 or selected from hydrogen, halogen atom, hydroxy group, mercapto group, amino group, nitro group, cyano group, aldehyde group, carbonyl group, carboxyl group, ester group, thioester group, isocyanate group, isothiocyanate group, epoxide group, cyclic carbonate group, cyclic thiocarbonate group, carbonate group, thiocarbonate group, amide group, sulfate group, sulphonate group, phosphonate group, sulfuryl group, sulfoxide group, aryl group, alkyl group, or modified alkyl group,
the active double bond is expressed by structural formula (XI) below: 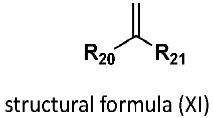
in structural formula (XI), R20 is further linked with the polymer chains or the micro/nano particles, and R18 before linked is one of the following end groups: end amino group, hydroxy group, mercapto group, halogen atom, acyl halide group, acid anhydride group, carboxyl group, carboxylate group, active ester group, isocyanate group, isothiocyanate group, epoxide group, cyclic carbonate group, cyclic thiocarbonate group, carbonate group, aryl group modified with active double bond group or active carbonyl group, heteroaromatic group, alkyl group, or modified alkyl group,
in structural formula (XI), R21 is either cyclized with R20 or selected from hydrogen, halogen atom, hydroxy group, mercapto group, amino group, nitro group, cyano group, aldehyde group, carbonyl group, carboxyl group, ester group, thioester group, isocyanate group, isothiocyanate group, epoxide group, cyclic carbonate group, cyclic thiocarbonate group, carbonate group, thiocarbonate group, amide group, sulfate group, sulphonate group, phosphonate group, sulfuryl group, sulfoxide group, aryl group, alkyl group, or modified alkyl group.

3. The medical tissue adhesive according to claim 1, **characterized in that**:
the dispersing agent is a liquid dispersing agent, comprising one of or a mixture of polyhydric alcohols, liquid polyethylene glycol and its derivatives.

4. The medical tissue adhesive according to claim 3, **characterized in that**:
the general formula of the polyhydric alcohols is CₙH₂ₙ₋ₘ₊₂(OH)ₘ, wherein n is positive integer greater than or equal to 2, m is positive integer less than or equal to n,
the polyethylene glycol and its derivative is one of or a mixture of linear PEG, branched PEG, multi-arm polyethylene glycol homopolymer and copolymer, end-modified PEG, and its ionization products.

5. The medical tissue adhesive according to claim 1, **characterized in that**:
the dispersing agent is solid dispersing agent, wherein the solid dispersing agent melts and evenly disperses the functional component and the assistant crosslinker upon heating, yet does not react with the functional component and the assistant crosslinker,
the dispersing agent is one of or a mixture of solid PEG, its derivatives, and saccharide chemicals.

6. The medical tissue adhesive according to claim 5, **characterized in that**:
the solid polyethylene glycol and its derivative is one of or a mixture of linear PEG, branched PEG, multi-arm polyethylene glycol homopolymer and copolymer, end-modified PEG, and its ionization products,
the saccharide chemicals is one of or a mixture of glucose, sucrose, maltose, xylitol, sorbitol.

7. The medical tissue adhesive according to claim 1, **characterized in that**:
the assistant crosslinker is the chemicals that react with the tissue-adhering functional groups, the assistant crosslinker is one of or a mixture of chemicals that bearing functional group comprising: amino group, carboxyl group, mercapto group, or hydroxy group.

8. The medical tissue adhesive according to claim 7, **characterized in that**:
the chemicals that bearing amino group is one of or a mixture of polyethyleneimine, polylysine, polyarginine, polyhistine, gelatin, collagen, elastin, fibroin, chitosan and its derivatives or copolymer, linear polyethylene glycol end-capped with amino group, multi-arm polyethylene glycol end-capped with amino group, and polyurethane end-capped with amino group,
the chemicals that bearing carboxyl group is one of or a mixture of hyaluronic acid, carboxymethyl chitosan, carboxymethyl cellulose, carboxymethyl starch, alginate, chondroitin sulfate, heparin, polyuronic acid, polyglutamic acid, polyaspartic acid, gelatin, collagen, fibroin, and their derivatives or copolymer,
the chemicals that bearing mercapto group is one or a mixture of polycystaine, gelatin, collagen, and their derivatives or copolymer,
the chemicals that bearing hydroxy group is one or a mixture of hyaluronic acid, chitosan, agarose, cellulose, starch, alginate, chondroitin sulfate, heparin, polyuronic acid, cyclodextrin, polyserine, gelatin, collagen, fibroin, polyvinyl alcohol, and their derivatives or copolymer.

9. The medical tissue adhesive according to claim 1, **characterized in that**:
the assistant crosslinker is a chemical that stimulates self-crosslink between the tissue-adhering functional group,
the assistant crosslinker is peroxide or reductant.

10. The medical tissue adhesive according to claim 9, **characterized in that**:
the peroxide is one or a mixture of alkyl peroxide, dialkyl peroxide, diacyl peroxide, ester peroxide, peroxydicarbonate, keto peroxide,
the reductant is one or a mixture of sulfite, hydrogen sulfite, ferrite, naphthenate, tertiary amine, and thiol alcohol.

11. The medical tissue adhesive according to claim 1, **characterized in that**:
the polymer chain is natural saccharide chemical, hydrophilic polymer, or water-soluble synthetic polymer.

12. The medical tissue adhesive according to claim 1, **characterized in that**:
the micro/nano particle is a nanoparticle or microparticle of a polymer chain,
the polymer chain is natural saccharide chemical, hydrophilic polymer, or water-soluble synthetic polymer.

13. The medical tissue adhesive according to claim 11 and claim 12, **characterized in that**:
the natural saccharide chemical is one or a mixture of hyaluronic acid, carboxymethyl cellulose, methyl cellulose, hydroxyethyl cellulose, hydroxypropyl cellulose, alginate, dextran, agarose, heparin, chondroitin sulfate, ethylene glycol chitosan, propylene glycol chitosan, chitosan lactate, carboxymethyl chitosan, and quaternary ammonium salt of chitosan.

14. The medical tissue adhesive according to claim 11 and claim 12, **characterized in that**:
the hydrophilic polymer or water-soluble synthetic polymer is one or a mixture of linear PEG, multi-arm PEG, polyethyleneimine, dendrimer, synthetic polypeptide, polyurethane, polylysine, polyglutamate, polyacrylate, polymethylacrylic acid, polymethacrylate, polyacrylamide, polymethacrylamide, polyvinyl alcohol, polyvinylpyrrolidone.

15. The medical tissue adhesive according to claim 1, **characterized in that**:
the mass ratio of the functional component, the assistant crosslinker, and the dispersing agent is 1:(0.01^{~}10):(0.1^{~}30).

16. A method to fabricate any of the medical tissue adhesive according to claim 1-15, **characterized in that** the process comprising:
grind the functional component and the assistant crosslinker into powder, mix the obtained powder with liquid dispersing agent evenly, and then the medical tissue adhesive is obtained,
or
grind the functional component and the assistant crosslinker into powder, mix the obtained powder with hot-melt dispersing agent evenly, and then the medical tissue adhesive is obtained.
